# EUROPEAN PATENT SPECIFICATION

(11) **EP 4 171 455 B1**
(45) Date of publication and mention of the grant of the patent: **11.12.2024**
(21) Application number: 21746577.2
(22) Date of filing: 21.06.2021
(51) Int. Cl.: A61F 9/007

(54) **INSTRUMENT FOR THE SURGICAL TREATMENT OF CATARACT, AIMED AT EXTRACTING THE NUCLEUS OF THE CRYSTALLINE LENS**
INSTRUMENT ZUR CHIRURGISCHEN BEHANDLUNG VON KATARAKT, DAS AUF DIE EXTRAKTION DES KERNS DER KRISTALLINEN LINSE ABZIELT
INSTRUMENT DESTINÉ AU TRAITEMENT CHIRURGICAL DE LA CATARACTE, VISANT À EXTRAIRE LE NOYAU DU CRISTALLIN

(30) Priority: 24.06.2020 IT 202000015139
(43) Date of publication of application: 03.05.2023
(73) Proprietor: STUDIO OCULISTICO DR.SSA ANTONIETTA CARBONE S.N.C., 96100 Siracusa (IT)
(72) Inventor: MUNNO, Ferdinando, 96100 - Siracusa (SR) (IT)
(74) Representative: Randazzo, Luigi
(86) International application number: PCT/IT2021/050192
(87) International publication number: WO 2021/260748

(56) References cited:
- WO-A2-2017/131930
- WO-A2-2017/161062
- US-A1- 2013 131 688

## Description

### • TECHNICAL FIELD

The present invention relates to ophthalmic surgical instruments and in particular to those used in cataract surgery.

### • BACKGROUND ART

As known, the cataracts is a typical eye disease of the elderly which consists in the opacification of the crystalline lens, which causes a progressive reduction of the visual capacity up to blindness.

The most frequent cause of cataract is aging, but it can also derive from eye trauma, ophthalmic or systemic diseases, hereditary or congenital defects. In the first case, more widespread, we speak of senile cataract caused by the hardening and slow yellowing of the fibers of the crystalline.

The cataract removal operation consists in the removal of the nucleus of the opacified crystalline and its subsequent replacement with a special artificial lens, suitable for the patient' s characteristics.

Currently, this operation is carried out according to a technique called "Facoemulsification", through which, at first, the membrane that surrounds the crystalline, i.e. capsular bag, is opened, then the crystalline nucleus is fragmented using an ultrasonic probe, and finally fragments of the nucleus and cortical masses of the opacified crystalline are sucked in.

The technique involves an incision of about 2 mm on the cornea (in the anterior part of the eye), the opening of the capsular bag through special pliers (capsuloressi), the detachment of the nucleus from its envelope through the input of water into the bag (hydrodissection). The procedure is completed with the fragmentation of the nucleus with a special instrument, the facoemulsificator, throughthe use of ultrasound and its subsequent aspiration. Finally, a particular intraocular lens is implanted that also corrects the visual defect of the patient, graduated and studied for each individual patient.

The greatest risk of the intervention executed with the current technique is the lesion or irreversible damage of the micrometric structures of the capsular bag that may occur during the phase of fragmentation of the nucleus' s crystalline lens inside it.

In this phase, in fact, the ultrasound emitted by the phaeomulsificator can cause the rupture of the posterior capsule of the crystalline lens, formed by a micrometric structure just 4 microns thick (a human hair measures about 10 times more than the crystalline capsule).

Complications related to the posterior capsule rupture consist in the fall of nucleus fragments into the vitreous chamber, that is the space between the posterior surface of the crystalline and the retina.

These unpleasant complications, besides compromising the success of the operation, do not allow the regular positioning of the artificial lens in its intended location (capsular bag) and must be implanted in alternative locations with risks of falling of very small parts into the vitreous chamber of the eye. All this can involve a risk for the regular recovery of vision, a risk of inaccuracy in the calculation of artificial lens to be implanted, post-operative blurring up to chronic inflammation. The fall of fragments of the nucleus of the original crystalline lens, moreover, makes necessary and urgent a new intervention of removal of said fragments through the so-called "vitrectomy", with new risks for the retina and for the general health of the eye. Another possible complication attributable to the current technique is the incomplete extraction of the crystalline lens that is achieved following the failure to recover a microfragment of the crystalline lens by the aspirator because the microfragment appears to have hidden behind elements anatomical that could be damaged by suction and suction. Also in this case, a subsequent intervention must be carried out to remove the microfragment.

In rarer cases, finally, corneal burns may occur due to the lack of cooling of the phaeomulsificator in the fragmentation phase. You have also consider the medical-legal problems and disputes concerning intra-operative eye damage essentially linked to the consequences of the rupture of the posterior capsule of crystalline.

On the market there are currently alternative techniques, especially used in Oriental Countries, where the availability of equipment that exploit ultrasound are limited. These techniques, however, provide the opening of the eye with clean cuts of more than 3 cm and their subsequent sutures to close them. These are extremely bloody interventions for the eye, also characterized by the onset of many complications. In this type of surgery a scleral incision is made on the eye and then a lesion of the crystalline capsule, which is removed in block without ultrasound with dedicated instruments. The drawback is that it is made a cut of several centimeters, with subsequent suture of the eye and with slower shooting times.

Prior art document US 2013/131688, discloses a medical device for use in endoscopy and polypectomy that performs the dual functions of both a severing or resection of a gastrointestinal (or "GI") polyp from the gastrointestinal tract wall and a trapping and retrieval of the gastrointestinal polyp from the gastrointestinal tact.

The invention is defined by the appended claims.

### • DISCLOSURE OF INVENTION

The invention in question, according to the claims, would introduce and allow a new technique in the ophthalmological field based on the complete removal of the nucleus of the crystalline lens instead of the current technique of fragmentation of the nucleus' s crystalline lens.

The surgical instrument, according to the found, introduces a technique that does not deviate from the current one in the early stages, as it will always proceed to the incision of the cornea, to the opening of the capsular bag through the special plier and finally to the hydrodissection of the nucleus. In this step, however, we will not proceed to the fragmentation and aspiration by the facoemulsificator, but removing the nucleus by the surgical instrument object of the present invention.

The surgical instrument, according to the found, is inserted inside the eye through the incisions made during the first phases of the surgery, thus allowing to arrive the height of the capsular bag. At this point, through an insertion mechanism that is operated by the handle, a bio-compatible collection chamber emerges, which will wrap the entire nucleus of the crystalline lens, leaving the capsular bag unharmed, through a circular gripping mechanism. The whole movement will not generate any pressure inside the eye but will be part of a regular surgical process, facilitated in the maneuvers by the introduction of a viscoelastic substance.

Through of a circular grip movement, in fact, the nucleus will be wrapped in the collection chamber of the instrument that is intended to be patented. Through the incorporation of the nucleus in the collection chamber of the surgical instrument is obtained the subsequent removal of the same without the use of the facoemulsificator. This avoids the use of ultrasound and the onset of countless related complications. Then proceed to the closing of the collection chamber with hermetic closing cursor. In this phase we proceed with the development of the fixing device inside the collection chamber with the aim of weakening the internal fibers of the crystalline lens. The surgical maneuver ends with the withdrawal of the collection chamber and the fixing device in its initial housing, removing the crystalline lens without fragmenting it as a result.

The surgery always ends, as expected by the current technique, with the implantation of an intraocular lens in the capsular bag.

The mechanism of this invention could be assimilated to that currently used in the technique of abdominal laparoscopy for the removal of the gallbladder: through this technique it is possible to remove large organs through incisions on the abdomen of just 2 cm. The organs, once freed from the vascular and nervous constraints, are placed in a special "pocket", sealed and removed from the organism.

In the same way, the surgical instrument according to the found aims to incorporate the nucleus of the crystalline lens after the detachment of the capsular bag from its involucre (then, after the phase of hydrodissection), and to remove it through the incisions practiced on the cornea.

The aim of this instrument is to reduce definitively the incidence of intraoperative complications related to cataract intervention by eliminating, in fact, the use of the facoemulsificator, a very expensive instrument.

In particular, the invention aims to eliminate the phase of fragmentation of the nucleus's crystalline lens inside the eye, avoiding the risk of the occurrence of the aforementioned complications and allowing the definitive removal of the nucleus of the crystalline lens, since this an instrument designed to incorporate the nucleus of the crystalline lens and extract it intact from the eye. It therefore avoids the use of ultrasound and the onset of countless related complications, such as micro-lesions or damage produced to the capsular bag in the phase of fragmentation.

This pourpose is achieved by the surgical instrument according to the first claim attached.

Another purpose of this invention is to design a surgical tool which, in addition to meeting the above purpose, can be easily sterilized after being used on a patient, so that it can be used for another patient.

Other advantageous characteristics are subject of the alleged claims, which are considered an integral part of this text.

The aforementioned purposes are achieved through the present found, in accordance with the claim n.1, which has an approximate weight of about 30 grams and is composed of:
- a rotating plastic handle of tubular shape that operates an insertion mechanism;
- an intermediate element, that is an elongated body of tubular shape with open sections, joined to one end of handle end and equipped with two channels inside it, designed to contain respectively:
   - a nitinol cable (or other deformable biocompatible metal alloy);
   - a fixing device, used to weaken the nucleus' s internal fibres;
- an collection chamber of oval shape, made of silicone material;
- a cursor adapted for hermetically closing of the collection chamber.

### • BRIEF DESCRIPTION OF DRAWINGS

Further characteristics and advantages of the invention will be more evident by means the detailed description of a preferred but not exclusive form of use, illustrated as a non-exhaustive example with reference to the attached drawings in which the figures show:

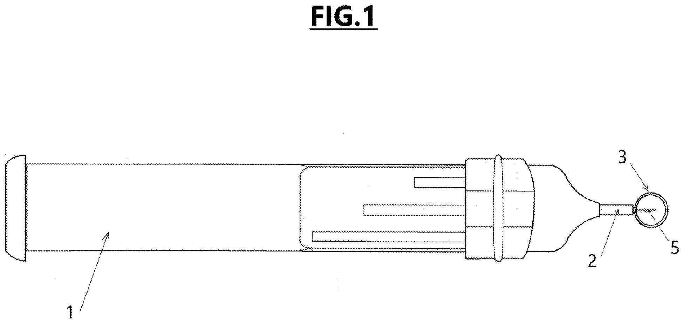

Figure 1: the whole instrument in prospectus, in view from above, in which are distinguished: the handle, the intermediate element and the cable;

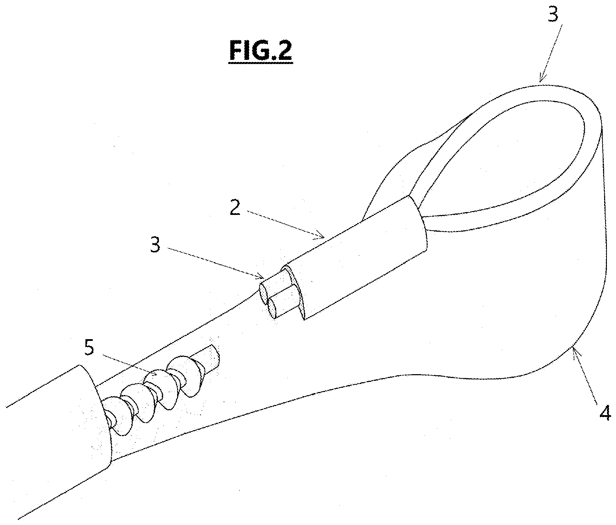

Figure 2: the intermediate element of the instrument, in view the exploded axonometric, in which are distinguished: the fixing device, cable and collection chamber;
Figure 3: a portion of the intermediate element of the instrument in longitudinal section in which are distinguished: the cable, the collection chamber, the cursor and the fixing device when the instrument is in closed mode.
Figure 4: a portion of the intermediate element of the instrument in view from above, when the instrument is in closed mode;
Figure 5: a portion of the intermediate element of the instrument in longitudinal section, during the withdrawal phase of the cable and of the collection chamber;
Figure 6: a portion of the intermediate element of the instrument in view from above, during the withdrawal phase of the cable and the collection chamber;
Figure 7: a portion of the intermediate element of the instrument in longitudinal section, during the sliding phase of the cursor along the two branches of the cable;
Figure 8: a portion of the intermediate element of the instrument in view from above, during the sliding phase of the cursor along the two branches of the cable; Please note: the fixing device is still inside the intermediate element;
Figure 9: a portion of the intermediate element of the instrument in longitudinal section, in the phase of progressive development of the fixing device;
Figure 10: a portion of the intermediate element of the instrument in view from above, in the phase of progressive development of the fixing device inside the collection chamber;
Figure 11: a portion of the intermediate element of the instrument in longitudinal section, during the withdrawal of the fixing device and subsequent withdrawal of the cable, the cursor and the collection chamber inside the intermediate element;
Figure 12: a portion of the intermediate element of the instrument in view from above, during the withdrawal of the fixing device and subsequent return of the cable, the cursor and the collection chamber within the intermediate element;

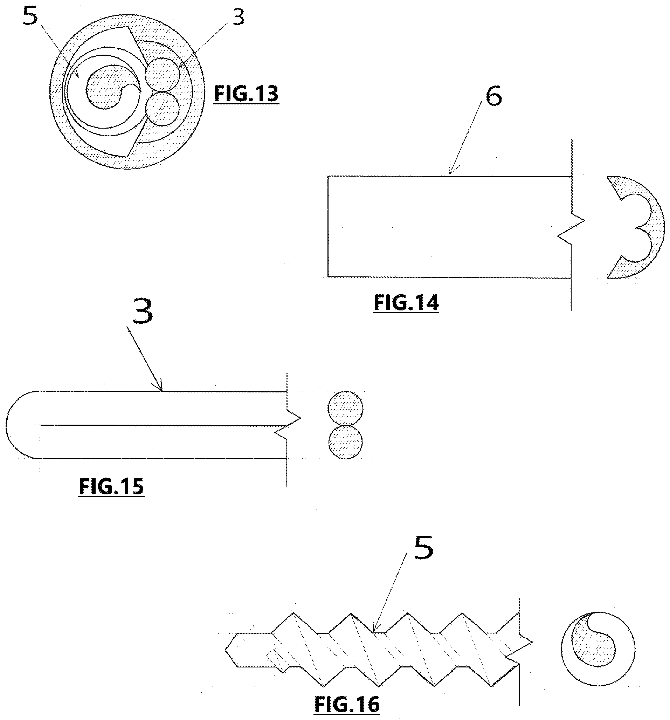

Figure 13: a cross section of the intermediate element of the instrument, in which are distinguished the cable, the cursor and the fixing device ;
Figure 14: a detailed view of the cursor, precisely: in prospectus and in cross section;
Figure 15: a detailed view of the cable, when the instrument is in closed mode, precisely: in prospectus and in cross section.
Figure 16: a detailed view of the fixing device, precisely: in prospectus and in cross section.

### • DESCRIPTION OF AT LEAST ONE WAY OF CARRYNG OUT THE INVENTION

The present invention will now be described by way of example and not limiting, according to a preferred form of implementation, also referring to the described figures that show the instrument in question in a progressive sequence. The purpose, as exposed, is to insert the instrument into the inner cavity of the eye, more precisely in the anterior chamber through an incision of 2.75 mm. A viscoelastic substance is then inserted with the effect of filling the spaces previously occupied by the aqueous mood.

We proceed first with the capsuloressi, that is opening of the anterior capsule of the crystalline lens with dedicated plier and then with hydrodissection, that is insertion of water between the capsule of the crystalline lens and its nucleus in order to unstick the two structures.

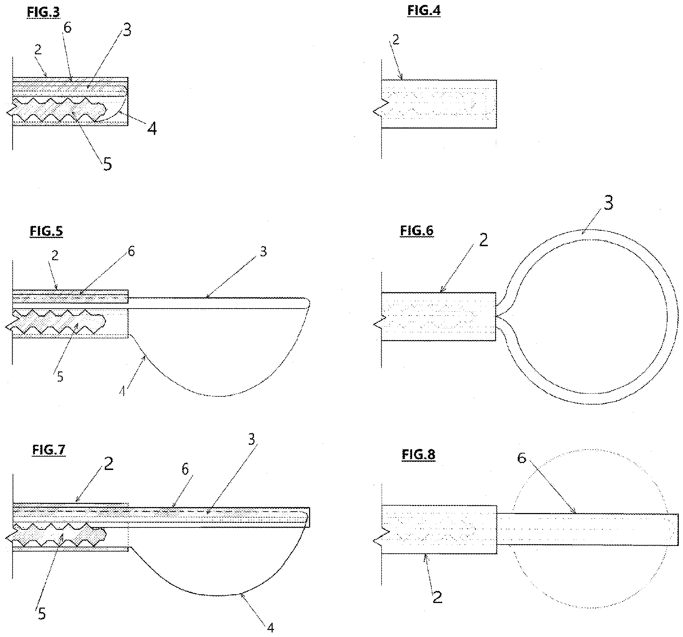

Now proceed with the insertion in the eye of the instrument according to the found (Fig. 3-4).

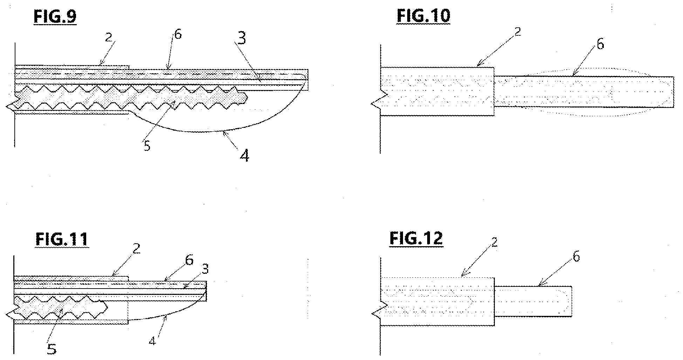

With a rotation maneuver of the handle 1 we obtain the development along the intermediate element 2 of cable 3 and, therefore, the escape of the collection chamber 4 and subsequent maneuver of collection and circular grip of the nucleus's crystalline lens, carried out by the surgeon (Fig. 5-6). This maneuver would be facilitated by the use of a viscoelastic substance. Then the surgeon, following the insertion of the nucleus's crystalline lens inside the collection chamber 4 of the instrument, proceeds to the hermetic closure of the collection chamber 4 by sliding the closing cursor 6 along the branches of cable 3 (Fig. 7-8). Through of the rotation mechanism of the handle 1, it is possible to obtain a progressive development of the fixing device 5, which weakens the fibres of the nucleus inside the collection chamber 4 (Fig. 9-10).

With an opposite rotation maneuver of handle 1 is obtained the withdrawl of the fixing device 5 inside the intermediate element 2, and hermetic closing with cursor 6 of the collection chamber 4 and contextual folding of the collection chamber 4 inside of the intermediate element 2 (Fig. 11-12).

At this point the instrument can be extracted from the eye.

From what is described it is evident that the instrument according to the invention achieves the predetermined aims.

The object of the invention is susceptible to numerous modifications and variants of a practical-applicative nature, all falling within the inventive concept expressed in the claims alleged. All the details may be replaced by other technically equivalent elements, and the materials may be different according to the needs, without leaving the scope of protection of this invention.

Although the object has been described with particular reference to the attached figures, the reference numbers used in the description and claims are used to improve the intelligence of the invention and do not constitute any limitation to the claimed scope of protection.

## Claims

1. Instrument for ophthalmic surgery and in particular for the surgical treatment of cataracts, adapted to remove the nucleus of the eye' s crystalline lens, in its integrity, without the use of ultrasound and without fragmentation thereof, comprising a tubular handle (1) which can be rotated in both directions which to control an insertion mechanism acting on a number of movable members of the instrument, respectively:
- a deformable metal alloy cable (3), folded to U,
- an oval-shaped collection chamber (4) of biocompatible material with an open section,
- a cursor (6) that surrounds at least part of the branches of the cable (3),
- a fixing device (5) comprising at least one screw, in which said movable members are contained in an immovable intermediate element (2), also shaped tubular with open sections,
**characterised by** the fact that:
- said cable (3), operated by said rotating handle (1), allows the output of the said collection chamber (4) adapted to wrap the nucleus of the crystalline lens,
- the sliding of the said fixing device (5), operated by the said rotating handle (1), is intended to weaken the internal fibres of the nucleus' s crystalline lens after having been incorporated in the collection chamber (4),
- the intermediate element (2) is equipped with at least two channels, adapted of receiving inside the metal alloy cable (3), joined to the collection chamber (4), in the upper channel and the said fixing device (5) in the lower channel,
- the said cursor (6), operated by this rotating handle (1) is intended to slide along the said branches of the cable (3), allowing the sealing of the said collection chamber (4),
- the said rotating handle (1) operated in the reverse anti-clockwise allows the withdrawal of these movable members.

2. Instrument according to the claim n.1, **characterized by** the fact that the said handle (1) operated to an insertion mechanism.

3. Instrument according to claim n.1, **characterized by** the fact that said intermediate tubular element (2) is joined to the end of the handle (1).

4. Instrument according to the claim n.1, **characterized by** the fact that the said metal alloy cable (3) and the said collection chamber (4) move in dependence to the rotations of the handle (1), with the possibility of assuming two different configurations: a first rest configuration, where the said branches of the the cable (3), abuts each other, are arranged entirely within the said intermediate element (2), and a second operational configuration in which the said branches of the cable (3) escaping from the said intermediate element (2) enveloped the edges of the inlet section of the collection chamber (4).

5. Instrument according to the claim n.1, **characterized by** the fact that the said collection chamber (4) joined to the said cable (3), can take at least two different configurations: a first rest configuration in which it is folded inside the said upper channel of the intermediate element (2) and a second operating configuration in which it comes out, allowing the said open section to wrap and to incorporate the nucleus of crystalline lens.

6. Instrument according to the claim n.1, in which this fixing device (5) comprising at least one screw is dependent on rotations of the handle (1), with the possibility of assuming two different configurations: a first rest configuration in which it is located within the said lower channel of the said intermediate tubular element (2) and a second operating configuration in which it coming out of the said intermediate element (2), slinding inside the collection chamber (4).

7. Instrument according to the claim n.1 in which said cursor (6) is **characterized by** the fact that it can take two different configurations: a first rest configuration in which it houses the said branches of the cable (3) inside the upper channel of the said intermediate tubular element (2) and a second operating configuration in which it slides along the said branches of the cable (3) and cooperates with them in such a way as to seal the collection chamber (4).

8. Instrument according to the claim n. 1, n. 2, n. 3, n. 4, n. 5, n. 6, n. 7, **characterized by** the fact that said insertion mechanism is designed to allow at least four distinct and progressive rotation steps: a first rotation step that allows the cable (3) joined to the collection chamber (4) to comes out of the said intermediate element; a second rotation step in which this cursor (6) comes out of the said intermediate element (2) by sliding along the said branches of the cable (3), a third rotation step that allows the fixing device (5) to comes out of the said intermediate element (2) and a fourth step of opposite rotation adapted to withdraw of the said movable members within the intermediate tubular element (2), returning to their rest configuration.

## Patentansprüche

1. Instrument für die Augenchirurgie und insbesondere für die chirurgische Behandlung von Katarakten, geeignet für die vollständige Entfernung des Kerns der Augenlinse ohne Verwendung von Ultraschall und ohne Fragmentierung derselben, bestehend aus einem drehbaren rohrförmigen Griff (1). in beide Richtungen, wodurch ein rotierender Einführungsmechanismus aktiviert wird, der jeweils auf mehrere bewegliche Elemente des Instruments wirkt:
- ein U-förmig gefaltetes Kabel (3) aus verformbarer Metalllegierung,
- eine ovale Sammelkammer (4) aus biokompatiblem Material mit offenem Abschnitt,
- einen Schieber (6), der die Abzweigungen des Kabels (3) zumindest teilweise umschließt,
- eine Befestigungsvorrichtung (5), bestehend aus mindestens einer Schraube,
wobei die beweglichen Elemente in einem unbeweglichen Zwischenelement (2) enthalten sind, das ebenfalls rohrförmig ist und offene Abschnitte aufweist, **dadurch gekennzeichnet, dass**:
- das Kabel (3), das durch den drehbaren Griff (1) aktiviert wird, ermöglicht den Austritt aus der Sammelkammer (4), die zur Aufnahme des Linsenkerns geeignet ist,
- das Abwickeln der Befestigungsvorrichtung (5), das durch den drehbaren Griff (1) betätigt wird, soll dazu dienen, die inneren Fasern des Linsenkerns zu schwächen, nachdem sie in die Sammelkammer (4) eingebracht wurden,
- das Zwischenelement (2) ist mit mindestens zwei Kanälen ausgestattet, die dazu geeignet sind, im oberen Kanal das mit der Sammelkammer (4) verbundene Metalllegierungskabel (3) und im unteren Kanal die Befestigungsvorrichtung (5) aufzunehmen Kanal,
- der Schieber (6), der durch den drehbaren Griff (1) betätigt wird, soll entlang der Zweige des Kabels (3) gleiten und so den hermetischen Verschluss der Sammelkammer (4) ermöglichen.
- der drehbare Griff (1), der in umgekehrter Richtung/gegen den Uhrzeigersinn betätigt wird, ermöglicht das Herausziehen der beweglichen Elemente.

2. Instrument nach Anspruch 1, **dadurch gekennzeichnet, dass** der in beide Richtungen drehbare Griff (1) einen Einführmechanismus aktiviert.

3. Instrument nach Anspruch 1, **dadurch gekennzeichnet, dass** das Zwischenrohrelement (2) mit dem Ende des Griffs (1) verbunden ist.

4. Instrument nach Anspruch 1, **dadurch gekennzeichnet, dass** sich das Metallgeflechtkabel (3) und die Sammelkammer (4) abhängig von den Drehungen des Griffs (1) bewegen, wobei zwei verschiedene Konfigurationen eingenommen werden können: eine erste Ruhekonfiguration, in der die benachbarten Kabelzweige (3) vollständig innerhalb des Zwischenelements (2) angeordnet sind, und eine zweite Betriebskonfiguration, in der die Kabelzweige (3) aus dem Zwischenelement (2) austreten und die Kanten des Zwischenelements (2) umgeben Einlassabschnitt der Sammelkammer (4).

5. Instrument nach Anspruch 1, **dadurch gekennzeichnet, dass** die mit dem Kabel (3) verbundene Sammelkammer (4) zwei verschiedene Konfigurationen annehmen kann: eine erste Ruhekonfiguration, in der sie im oberen Teil des Kanals gefaltet ist das Zwischenelement (2) und eine zweite Betriebskonfiguration, in der es herauskommt, so dass der offene Abschnitt den Kern der Linse umhüllen und einschließen kann.

6. Instrument nach Anspruch 1, bei dem die Befestigungsvorrichtung (5), die mindestens eine Schraube umfasst, von den Drehungen des Griffs (1) abhängig ist, mit der Möglichkeit, zwei unterschiedliche Konfigurationen anzunehmen: eine erste Ruhekonfiguration, in der es ist innerhalb des genannten unteren Kanals des genannten rohrförmigen Zwischenelements (2) angeordnet und hat eine zweite Betriebskonfiguration, in der es aus dem genannten Zwischenelement (2) austritt und sich innerhalb der Sammelkammer (4) abwickelt.

7. Instrument nach Anspruch 1, bei dem der Schieber (6) **dadurch gekennzeichnet ist, dass** er zwei verschiedene Konfigurationen annehmen kann: eine erste Ruhekonfiguration, in der er die Zweige des Kabels (3) im oberen Kanal aufnimmt des Zwischenrohrelements (2) und einer zweiten Betriebskonfiguration, in der es nach außen vorsteht, entlang der Zweige des Kabels (3) gleitet und mit ihnen zusammenwirkt, um die Sammelkammer (4) hermetisch zu verschließen.

8. Instrument nach Anspruch Nr. 1, Nr. 2, Nr. 3, Nr. 4, Nr. 5, Nr. 6, Nr. 7, **dadurch gekennzeichnet, dass** der Einführmechanismus so gestaltet ist, dass er mindestens vier verschiedene Durchgänge ermöglicht Rotationsabläufe: ein erster Rotationsschritt, der es ermöglicht, dass das an der Sammelkammer (4) befestigte Kabel (3) aus dem Zwischenelement (2) austritt; einen zweiten Drehschritt, bei dem der Schieber (6) aus dem Zwischenelement (2) austritt und entlang der Zweige des Kabels (3) gleitet; ein dritter Drehschritt, der es der Befestigungsvorrichtung (5) ermöglicht, aus dem Zwischenelement (2) herauszukommen (2) und einen vierten Schritt der umgekehrten Drehung, der dazu dient, die beweglichen Elemente in das rohrförmige Zwischenelement (2) zurückzuziehen und in ihre Ruhekonfiguration zurückzukehren.

## Revendications

1. Instrument pour la chirurgie ophtalmique et en particulier pour le traitement chirurgical de la cataracte, adapté à l'ablation du noyau du cristallin, dans sa totalité, sans utilisation d'ultrasons et sans fragmentation de celui-ci, comprenant un manche tubulaire (1) rotatif dans les deux sens qui actionne un mécanisme d'insertion en rotation agissant respectivement sur une pluralité d'éléments mobiles de l'instrument:
- un câble (3) en alliage métallique déformable, plié en U,
- une chambre de collecte (4) de forme ovale en matériau biocompatible à section ouverte,
- un curseur (6) qui entoure au moins partiellement les branches du câble (3),
- un dispositif de fixation (5), constitué d'au moins une vis, dans lequel lesdits éléments mobiles sont contenus dans un élément intermédiaire immobile (2), également de forme tubulaire, à sections ouvertes, **caractérisé par le fait que**:
- ledit câble (3), activé par ladite poignée rotative (1), permet la sortie de ladite chambre de collecte (4) adaptée à l'incorporation du noyau de la lentille,
- le déroulement dudit dispositif de fixation (5), actionné par ladite poignée rotative (1), est destiné à fragiliser les fibres internes du noyau du cristallin après son incorporation dans ladite chambre de collecte (4),
- ledit élément intermédiaire (2) est équipé d'au moins deux canaux adaptés pour recevoir, à l'intérieur, ledit câble en alliage métallique (3) relié à ladite chambre de collecte (4) dans le canal supérieur, et ledit dispositif de fixation (5) dans le canal inférieur canal,
- ledit curseur (6) actionné par ladite poignée rotative (1) est destiné à coulisser le long desdites branches du câble (3), permettant la fermeture hermétique de ladite chambre de collecte (4),
- ladite poignée rotative (1) actionnée dans le sens inverse/anti-horaire permet le retrait desdits éléments mobiles.

2. Instrument selon la revendication n° 1, **caractérisé par le fait que** ladite poignée (1), qui peut tourner dans les deux sens, actionne un mécanisme d'insertion.

3. Instrument selon la revendication n° 1, **caractérisé par le fait que** ledit élément tubulaire intermédiaire (2) est solidarisé à l'extrémité du manche (1).

4. Instrument selon la revendication n° 1, **caractérisé par le fait que** ledit câble en treillis métallique (3) et ladite chambre de collecte (4) se déplacent en fonction des rotations du manche (1), avec possibilité de prendre deux configurations différentes : une première configuration de repos, dans laquelle lesdites branches de câble adjacentes (3) sont disposées entièrement à l'intérieur dudit élément intermédiaire (2) et une deuxième configuration de fonctionnement dans laquelle lesdites branches de câble (3) émergent dudit élément intermédiaire (2) entourent les bords du section d'entrée de la chambre de collecte (4).

5. Instrument selon la revendication n° 1, **caractérisé par le fait que** ladite chambre de collecte (4) reliée audit câble (3), peut prendre deux configurations différentes: une première une configuration de repos dans laquelle elle est repliée à l'intérieur dudit canal supérieur dudit élément intermédiaire (2) et une seconde configuration opérationnelle dans laquelle elle ressort, permettant à ladite section ouverte d'envelopper et d'incorporer le noyau de la lentille.

6. Instrument selon la revendication n.1, dans lequel ledit dispositif de fixation (5) comprenant au moins une vis est dépendant des rotations du manche (1), avec possibilité de prendre deux configurations différentes : une première configuration de repos dans laquelle il est disposé à l'intérieur dudit canal inférieur dudit élément tubulaire intermédiaire (2) et une deuxième configuration opérationnelle dans laquelle il sort dudit élément intermédiaire (2) en se dépliant à l'intérieur de la chambre de collecte (4).

7. Instrument selon la revendication n° 1 dans lequel ledit coulisseau (6) se **caractérise par le fait qu'**il peut prendre deux configurations différentes : une première configuration de repos dans laquelle il loge lesdites branches du câble (3) à l'intérieur du canal supérieur dudit élément tubulaire intermédiaire (2) et une deuxième configuration opérationnelle dans laquelle il fait saillie vers l'extérieur, coulissant le long desdites branches du câble (3) et coopère avec elles de manière à fermer hermétiquement la chambre de collecte (4).

8. Instrument selon la revendication n.1, n.2, n.3, n.4, n.5, n.6, n.7, **caractérisé en ce que** ledit mécanisme d'insertion est conçu pour permettre au moins quatre passages distincts et progressions de rotation: une première étape de rotation qui permet au câble (3) fixé à la chambre de collecte (4) de sortir dudit élément intermédiaire (2); une deuxième étape de rotation dans laquelle ledit curseur (6) sort dudit élément intermédiaire (2) en glissant le long desdites branches du câble (3), une troisième étape de rotation qui permet au dispositif de fixation (5) de sortir dudit élément intermédiaire (2) et une quatrième étape de rotation inverse destinée à obtenir le retrait desdits éléments mobiles à l'intérieur de l'élément tubulaire intermédiaire (2), retournant à leur configuration de repos.
